# EUROPEAN PATENT SPECIFICATION

(11) **EP 4 088 650 B1**
(45) Date of publication and mention of the grant of the patent: **05.03.2025**
(21) Application number: 21173319.1
(22) Date of filing: 11.05.2021
(51) Int. Cl.: A61B 5/00, A61B 5/107, G01R 33/48, G06T 7/136, G06T 7/00, G06T 7/62

(54) **METHOD OF QUANTIFYING AN EFFECTIVE VOLUME OF A MUSCLE**
VERFAHREN ZUR QUANTIFIZIERUNG EINES EFFEKTIVEN VOLUMENS EINES MUSKELS
PROCÉDÉ DE QUANTIFICATION D'UN VOLUME EFFECTIF D'UN MUSCLE

(43) Date of publication of application: 16.11.2022
(73) Proprietor: AMRA Medical AB, 582 22 Linköping (SE)
(72) Inventor: DAHLQVIST LEINHARD, Olof, 582 22 Linköping (SE)
(74) Representative: AWA Sweden AB

(56) References cited:
- EP-A1- 3 693 976
- WO-A1-2019/118353
- US-A1- 2017 046 837

## Description

### Technical Field

The present disclosure relates to a method of determining an effective volume of a muscle, and especially to a method using a three-dimensional water fat separated image of the muscle.

### Background

In medical evaluations of subjects, and especially when identifying muscle related conditions, it may be difficult to provide a relevant assessment of the condition of a muscle. Some muscle related conditions, such as sarcopenia, develop slowly over time, which may make commonly used methods, in which specific tests are repeated, difficult to use.

A muscle related condition may be characterized by progressive loss of muscle quantity and function over time associated to adverse outcomes in several different disease areas.

For identification of low muscle mass, dual-energy X-ray absorptiometry (DXA) and bioelectrical impedance analysis (BIA) are widely used to estimate appendicular skeletal muscle mass (ASM) and provide basis for identification of thresholds sensitive to subjects with particularly low muscle mass.

A major challenge in understanding a muscle related condition and its consequences is the wide variation in normal physiology present in the general population. The aetiology for low muscle quantity is naturally widespread; Certain phenotypes with low muscle quantity could be associated with longevity as a healthy lifestyle in combination with low-caloric intake may result in low muscle quantity. With higher body mass index (BMI), the muscle quantity increases as a result of the individual carrying an increasingly larger body weight, making the identification of patients with a muscle related condition by low muscle quantity more difficult. This have led to disclosed methods of applying a range of body size adjustments to measured muscle quantity, namely by dividing e.g. ASM with height, weight, or BMI. However, methods with such adjustments may be difficult to get reliable.

A method of quantifying a lean tissue volume is disclosed in US2017/0046837A1.

It is clear that muscle quantity and/or quality is an important measure when determining a muscle related condition of a subject. Consequently, due to the variations of subjects' bodies, there is a need for a method of determining the quantity and quality of a muscle that is reliable and that provides an effective measure of the condition of the muscle.

### Summary

It is an object of the present invention to provide an improved solution that alleviates the mentioned drawbacks with present devices. Furthermore, it is an object to provide method of quantifying an effective volume of a muscle.

The invention is defined by the appended independent claims, with embodiments being set forth in the appended dependent claims, in the following description and in the drawings.

According to the present invention, a method of quantifying an effective volume of a muscle of a subject, the method comprising the steps of acquiring a three-dimensional water and fat separated image of the muscle, said image comprising volume elements each having water and fat level values, determining a muscle fat infiltration level for each volume element in the acquired image of the muscle, determining a first volume region of the muscle in said image in which the muscle fat fraction, MFF1, is below a predetermined threshold level, T1, and calculating an effective volume representation of the muscle by multiplying the volume of the first volume region with WF(MFF1), wherein WF(MFF1) is a weighting function of the muscle fat fraction, MFF1, in said first volume region.

Magnetic resonance imaging (MRI), together with computed tomography (CT), is considered gold standard for non-invasive assessment of muscle quantity. Body composition profiling is a concept utilizing a standardized MRI examination enabling simultaneous assessment of traditional body composition (total fat-free muscle tissue volume and total adipose tissue volume), as well as detailed characterization of adipose tissue distribution and ectopic fat accumulation, such as visceral adipose tissue (VAT), liver fat and muscle fat infiltration (MFI). Muscle fat infiltration has previously been used as a quantitative measure of muscle quality and is a well-established biomarker in the description of different muscular dystrophies.

The three-dimensional water fat separated image may in one embodiment be determined using an MRI scan and image analysis of the MRI image may be used to determine the first volume region. The muscle may in one embodiment be a thigh muscle since the size of the thigh muscle may be closely related to the body size of the individual.

The volume elements of the three-dimensional water fat separated image may be voxels of the three-dimensional image. Image analysis may be performed to determine the muscle fat fraction level in the muscle. For each voxel of the muscle in the image, the fat level may be determined to establish the muscle fat fraction level for that voxel. The acquired image may be a full body scan or a partial body scan of the subject, with a region of interest defined of the muscle of interest. In one embodiment, the method may simultaneously be applied to more than one muscle represented in the acquired image.

When determining the first volume region, the fat fraction level for each voxel of the muscle is evaluated such that all voxels having a muscle fat fraction level below the predetermined threshold level together forms the first volume region. The predetermined threshold level may be predetermined based on characteristics of the subject. Such characteristics may for example be sex, age, or BMI.

By determining the first volume region it may be meant determining which voxels in the part of the acquired image representing the muscle that have a fat level below a threshold level corresponding to the predetermined threshold level of the muscle fat fraction. The first volume region may in one embodiment be formed strictly by all voxels having a fat level below the threshold level. Alternatively, the first volume region may in one embodiment be formed with regard to, besides the threshold level, the fat levels of voxels adjacent to a particular voxel.

To determine the effective volume of the muscle, a calculation is performed in which the volume of the first volume region is multiplied with a weighting function of the muscle fat fraction in the first volume region. The effective volume of the muscle is thereby based on the muscle fat fraction of the entire muscle, while a part of the muscle having a muscle fat fraction above the predetermined threshold level may be disregarded. Hence, the parts of the muscle having a muscle fat fraction level above the predetermined threshold is completely disregarded for the effective volume. The parts of the muscle in the first volume region is used as basis for the effective volume, but the muscle fat fraction levels in the parts forming the first volume region is used as a weight to the contribution such part provides to the function of the muscle. Such muscle part may be a voxel in the three-dimensional image. The function of a part of the muscle represented by a voxel may thereby be considered as zero when having a muscle fat fraction level above the predetermined threshold level, i.e. at a level where there still is muscle tissue left and not pure fat. Using the weighting function, the function of the muscle in a part represented by a voxel moves towards zero when the muscle fat fraction level increases towards the predetermined threshold level. The weighting function sets how the muscle function changes with an increasing muscle fat fraction level towards the predetermined threshold.

By determining the effective volume of the muscle, a quantification of the function of the muscle is provided that in a reliable and precise way represent the actual muscle function. The effective volume may be seen as a representation of a functional volume of the muscle. The effective volume may for instance be used in longitudinal monitoring of the muscle function when the muscle slowly loses function.

According to one embodiment, the predetermined threshold level, T1, of muscle fat fraction maybe between 30-80%, preferably between 45-55%, more preferably 50%. For each voxel of the acquired image forming the muscle, the predetermined threshold level may provide a predetermined fat level. For each voxel, it may be determined if the fat level is below the predetermined fat level and thereby forming part of the first volume region. The threshold level may in one embodiment be 50%, meaning that the first volume region may be formed by all voxels in the region of interest in the acquired image having a fat level of less than 50% of a maximum fat level. The maximum fat level may mean that the part of the muscle being represented by a particular voxel comprises 100% fat and 0% muscle tissue. The effective volume may thereby provide that all parts of the muscle having more than 50% of muscle fat fraction is considered to provide zero muscle function, and all parts of the muscle having less than 50% muscle fat fraction may provide muscle function at a level determined by the weighting function. The same may apply to other threshold levels in e.g. the interval of 30-80%.

According to one embodiment, the weighting function WF(MFF1) may be equal to 1 when MFF1 is 0 and wherein WF(MFF1) may decrease towards 0 when MFF1 increases towards T1. This may also be expressed as the weighting function being a monotonically decreasing function with WF(0) being equal to 1 and WF(T1) being equal to 0.

According to one embodiment, the weighting function WF(MFF1) may be a linear function of MFF1. A linear function of the muscle fat fraction, to which the first volume region is multiplied, may provide a basic relationship between the entities to determine the effective muscle volume. A linear weighting function may provide that, for the parts of the muscle having a muscle fat fraction level below the predetermined threshold level, the muscle function represented by the effective volume moves linearly towards zero with an increasing muscle fat fraction level towards the predetermined threshold level. In one embodiment, the weighting function WF(MFF1) may be 1-(MFF1/T1).

According to another embodiment, the weighting function WF(MFF1) may be a non-linear function of MFF1. A non-linear function of the muscle fat fraction may provide a more complex determination of the effective muscle volume, thereby providing further capabilities in determining a subject's muscle related condition. A non-linear weighting function may provide that, for the parts of the muscle having a muscle fat fraction level below the predetermined threshold level, the muscle function represented by the effective volume moves non-linearly towards zero with an increasing muscle fat fraction level towards the predetermined threshold level T1. With a non-linear decreasing effective volume with increasing muscle fat fraction, a representation of the muscle function may be provided that consider further aspects of the muscle function at different levels of muscle fat fraction. For instance, the closer the muscle fat fraction level gets to the predetermined threshold level T1, the larger effect it may have on decreasing that volume element's contribution to the effective volume. In one embodiment, the non-linear function may be exp(-k*(MFF1/T1)), where k is a positive integer. In another embodiment, the non-linear weighting function may be (1-MFF1/T1)².

According to one embodiment, the muscle fat fraction level may be determined for each volume element based on the water and/or fat level values for the respective volume element. A volume element may be represented by a voxel in the acquired water and fat separated image. The water level in the water image of the acquired image and the fat level in the fat image of the acquired image may together provide a muscle fat fraction value for a particular voxel. The acquired image may be normalized such that a fat level value of 1, or 100%, represents a volume element of pure fat without any muscle tissue. The muscle fat fraction in a voxel with a fat level of 1 or 100% may then be 100%. In a voxel having a fat level of 0, the muscle fat fraction may be 0%. The predetermined threshold level, T1, may be related to the fat level in a voxel. A predetermined threshold level of 50% may correspond to a fat level in a voxel of 50% of a maximum fat level, e.g. 1 or 100%. When calculating the effective volume, the muscle fat fraction level for each volume element may provide a weighting function for each volume element to which the volume of the respective volume element is multiplied. The effective volume may then be provided by the sum of the calculated volumes for all volume elements of the first volume region.

According to one embodiment, the first volume region may be provided by the volume elements of the muscle having a MFF1 below the predetermined threshold level, T1. All voxels in the acquired image having a muscle fat fraction below the predetermined threshold level may then together form the first volume region. The volume of the first volume region may thereby be provided by the sum of the volumes of the volume elements constituting the first volume region. In one embodiment, whether a voxel is determined as part of the first volume region may further be based on the muscle fat fraction level of voxels adjacent to the present voxel. For instance, a particular voxel may only be determined to be part of the first volume region if one or more of adjacent voxels are above a certain level. Additionally, or alternatively, the muscle fat fraction level of a particular voxel may be determined or adjusted further based on muscle fat fraction levels of one or more adjacent voxels.

According to one embodiment, the effective volume of the muscle may provide a quantification of the part of the muscle having a functional level at a predetermined rate. In order to make a determination of a subject's functional level in a muscle, either on short-term or long-term, a determination of a representation of the effective volume of the muscle may be relevant to provide, which then may give a quantified representation of an actual function of the muscle.

According to one embodiment, the three-dimensional water fat separated image may be an MRI image.

According to a second aspect of the present invention, a system is provided configured to perform the method according to any of the embodiments above, the system comprising means for acquiring the three-dimensional water and fat separated image, and means configured to determine the muscle fat fraction level in the muscle, determine the first volume region, and calculate the effective volume. The system may comprise a computer device comprising a receiving unit configured to receive the image and a processor unit configured to determine the muscle fat fraction level, determine the first volume region and calculate the effective volume. The system may further comprise means and be configured to perform the methods according to any of the embodiments above.

### Brief Description of the Drawings

The invention will in the following be described in more detail with reference to the enclosed drawings, wherein:
Fig. 1 shows a flow chart of a method according to an embodiment of the invention;
Fig. 2 shows a schematic block view of a system according to an embodiment of the invention;
Fig. 3 shows a chart of a determination step according to an embodiment of the invention;
Fig. 4 shows a schematic view of a representation of a muscle in an embodiment of the invention;
Fig. 5 shows a schematic view of a first volume region determine on a representation of a muscle in an embodiment of the invention; and
Fig. 6 shows a schematic view of a first volume region in an embodiment of the invention.

### Description of Embodiments

The present invention will be described more fully hereinafter with reference to the accompanying drawings, in which preferred embodiments of the invention are shown. This invention may, however, be embodied in many different forms and should not be construed as limited to the embodiments set forth herein; rather, these embodiments are provided so that this disclosure will be thorough and complete, and will fully convey the scope of the invention to those skilled in the art. In the drawings, like numbers refer to like elements.

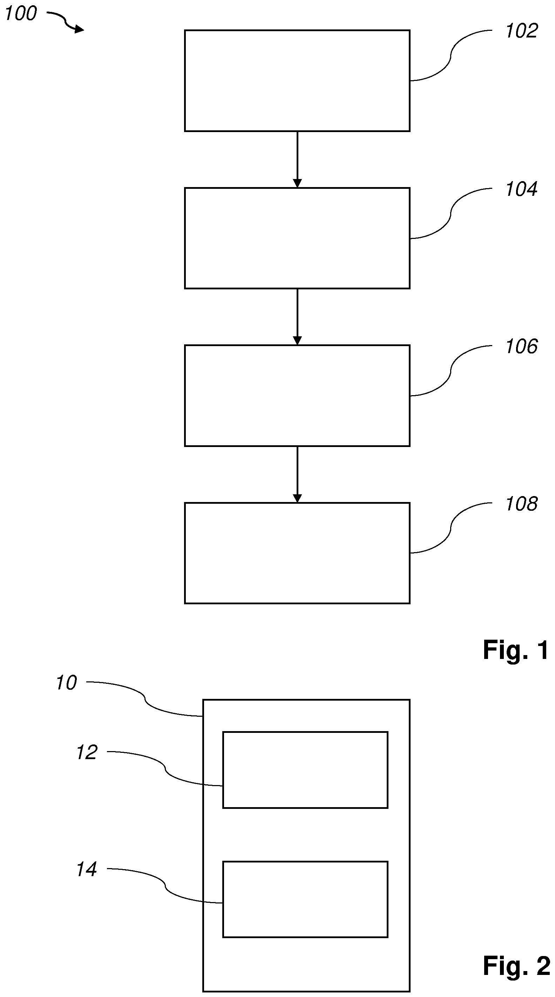

Fig. 1 illustrates a method 100 of determining an effective volume of a muscle 20 of a subject according to an embodiment of the present invention. The method 100 comprises a step 102 of acquiring a three-dimensional water fat separated image. The image may be acquired by an acquiring means 12 being part of a system 10 configured to determine an effective volume of a muscle 20 of a subject as illustrated in Fig. 2. The three-dimensional water fat separated image may be provided based on an MRI scan.

Next, based on the data in the three-dimensional water fat separated image, a determination 104 of a muscle fat fraction MFF1 in the muscle 20 is performed. The muscle fat fraction of the muscle 20 is provided by the water levels and the fat levels for each voxel in the water image and the fat image of the three-dimensional water fat separated image. From this step 104, a muscle fat fraction level for each voxel representing a three-dimensional portion of the muscle 20 in the acquired image is provided.

Based on a predetermined threshold level T1, a first volume region 30 is determined by determining 106, for each voxel, whether the muscle fat fraction MFF1 for a particular voxel is below the predetermined threshold level T1. All voxels for which the muscle fat fraction level MFF1 is below the threshold level T1 together form the first volume region 30.

In the final step of the method 100, a calculation 108 of an effective volume of the muscle 20 is performed. In the calculation 108, the volume of the first volume region 30 is multiplied with a weighting function WF(MFF1) being a function of the muscle fat fraction level MFF1 in the first volume region 30.

The steps of determining the muscle fat fraction, determining the first volume region 30, and calculating the effective volume may be performed by a means 14 configured thereto being part of the system 10, as illustrated in fig. 2. The means 12, 14 may be provided by a computer device.

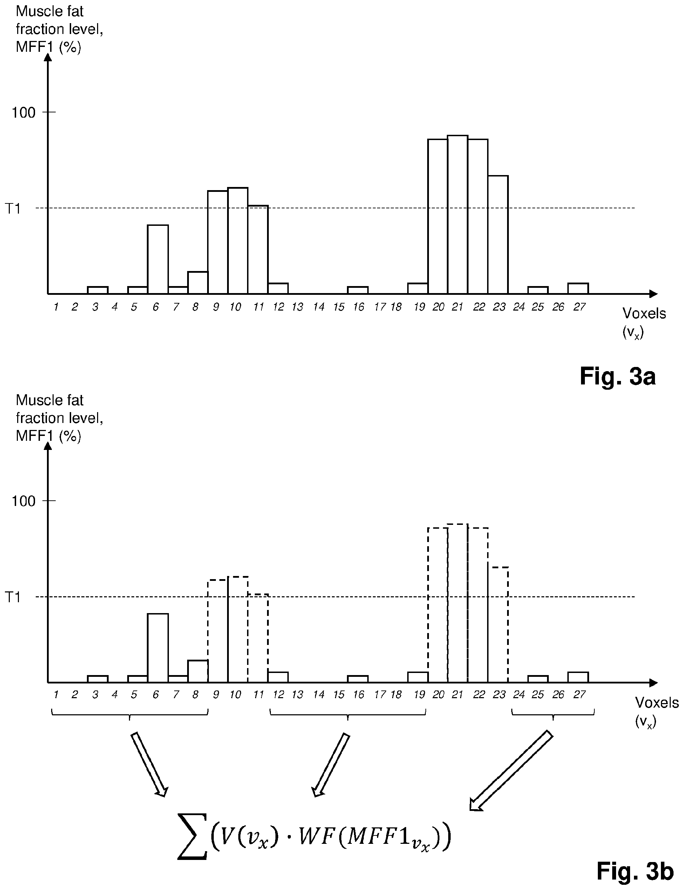

In Fig. 3a, a chart schematically illustrates the determination of the first volume region 30 by determining whether the muscle fat fraction level MFF1 for a particular voxel v₁-v₂₇ is below the predetermined threshold level T1. The voxels having a muscle fat fraction level below the threshold T1 together forms the first volume region 30. This is shown in fig. 3b, illustrating the determination of the effective volume VE. Voxels v₉-v₁₁ and v₂₀-v₂₃ are excluded due to a muscle fat fraction level above the threshold T1. The remaining voxels together form the first volume region 30. The volume of each voxel, V(vₓ), is multiplied with the weighting function WF for that particular voxel, WF(MFF1ᵥₓ). The weighting function WF for a particular voxel is based on that voxel's muscle fat fraction level MFF1ᵥₓ. As discussed above, the weighting function may in one embodiment be 1 for a MFF1 of 0 and 0 for a MFF1 of T1. In the illustrated embodiment of fig. 3b, voxels v₁, v₂, v₄, v₁₃-v₁₅, v₁₇, v₁₈, v₂₄ and v₂₆ the weighting function will be 1, providing that the entire volume of these voxels will be used for the effective volume VE. For the other voxels being part of the first volume region, the muscle fat fraction level MFF1ᵥₓ for each voxel will provide a reduction of the volume contributing to the effective volume VE based on the linear or non-linear weighting function WF. The effective volume VE is then determined by the sum of the calculations for all voxels being part of the first volume region 30. Hence, the effective volume may in one embodiment be determined by ∑(V(vₓ)·WF(MFF1ᵥₓ)).

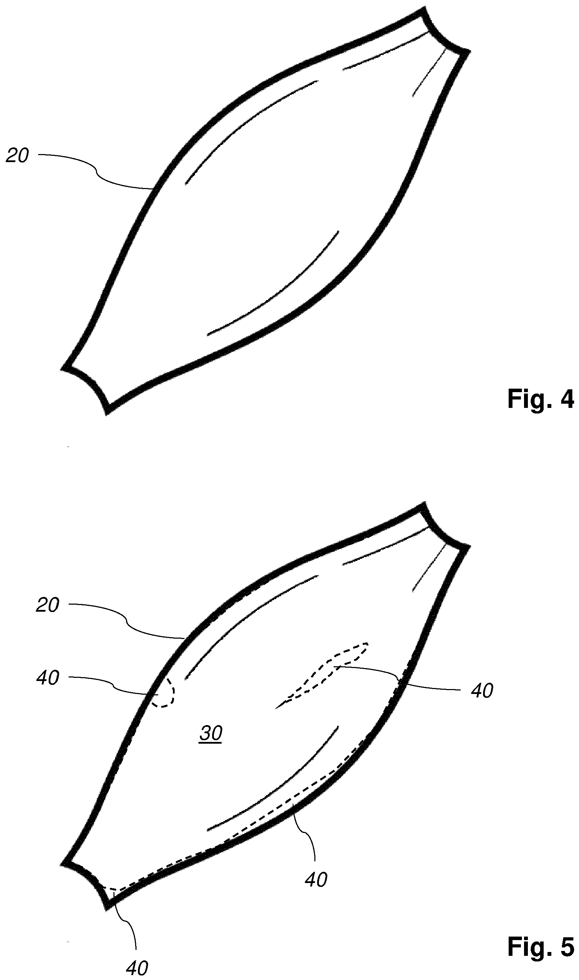

The first volume region 30 is further illustrated in Figs. 4 and 5. The muscle 20 is represented in the three-dimensional water fat separated image as seen in Fig. 4. After determination of the muscle fat fraction levels in the muscle 20, the first volume region 30 is determined as discussed above. The first volume region 30 is the entire volume of the muscle 20 minus the volume portions 40 having a too high muscle fat fraction level. In Fig. 5 this is illustrated by excluding volume portions 40 from the muscle volume 20, resulting in the first volume region 30. The first volume portion 30 may be a single continuous volume portion, or comprising two or more volume portions separated by one or more excluded volume portions 40.

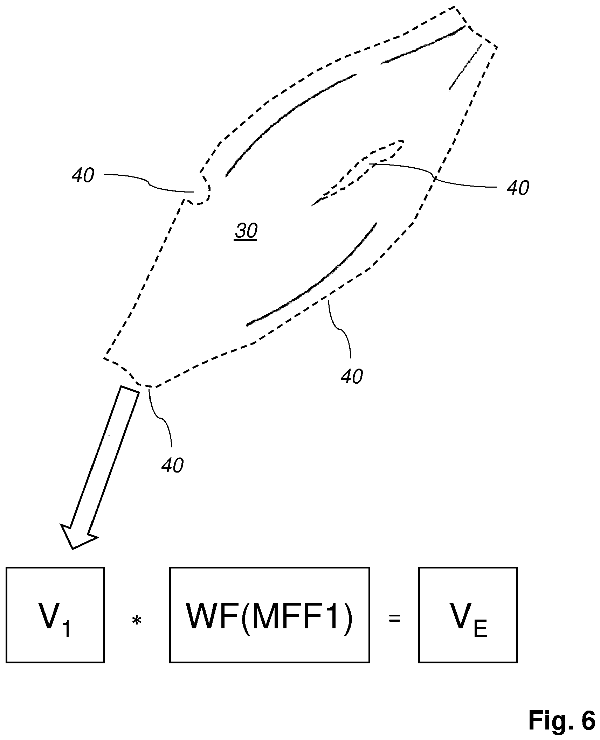

The resulting first volume region 30 from the muscle 20 in Fig. 5 is illustrated in Fig. 6. Based on the determined first volume region 30 and the voxels constituting the first volume region 30, the volume V₁ of the first volume region 30 is determined. The volume V₁ is then multiplied with the weighting function WF(MFF1) to determine the effective volume VE of the muscle 20. As discussed above, this may be provided by summing the calculations of each respective voxel being part of the first volume region 30.

The first volume region 30 excludes all voxels in the image of the muscle 20 that have a muscle fat fraction above the threshold T₁. In the effective volume VE, the voxels representing the muscle 20 contribute to the muscle function in a gradual manner based on the muscle fat fraction level in the particular voxel.

In the drawings and specification, there have been disclosed preferred embodiments and examples of the invention and, although specific terms are employed, they are used in a generic and descriptive sense only and not for the purpose of limitation, the scope of the invention being set forth in the following claims.

## Claims

1. A method (100) of quantifying an effective volume (V_{E}) of a muscle (20) of a subject, the method comprising the steps of:
acquiring (102) a three-dimensional water and fat separated image of the muscle, said image comprising volume elements each having water and fat level values,
determining (104) a muscle fat fraction level, MFF1, for each volume element in the acquired image of the muscle,
**characterized by**
further comprising the steps of:
determining (106) a first volume region (30) of the muscle in said image in which the muscle fat fraction level, MFF1, is below a predetermined threshold level, T1,
calculating (108) an effective volume representation of the muscle by multiplying the volume of the first volume region with WF(MFF1), wherein WF(MFF1) is a weighting function of the muscle fat fraction, MFF1, in said first volume region.

2. The method according to claim 1, wherein the predetermined threshold level, T1, of muscle fat fraction is between 30-80%, preferably between 45-55%, more preferably 50%.

3. The method according to claim 1 or 2, wherein the weighting function WF(MFF1) further is a function of the predetermined threshold level, T1.

4. The method according to any of the preceding claims, wherein the weighting function WF(MFF1) equals 1 when MFF1 is zero and decreases towards 0 when MFF1 increases towards T1.

5. The method according to any of the preceding claims, wherein the weighting function WF(MFF1) is a linear function of MFF1.

6. The method according to claim 4, wherein the weighting function WF(MFF1) is 1-(MFF1/T1).

7. The method according to any of the claims 1-4, wherein the weighting function WF(MFF1) is a non-linear function of MFF1.

8. The method according to claim 7, wherein the weighting function WF(MFF1) is exp(-k*(MFF1/T1)), where k is a positive integer.

9. The method according to claim 7, wherein the weighting function WF(MFF1) is (1-MFF1/T1)².

10. The method according to any of the preceding claims, wherein the muscle fat fraction level is determined for each volume element based on the water and fat level values for the respective volume element.

11. The method according to any of the preceding claims, wherein the first volume region (30) is provided by the volume elements of the muscle (20) in the image having a MFF1 below the predetermined threshold level, T1.

12. The method according to any of the preceding claims, wherein the effective volume (30) of the muscle (20) provides a quantification of the part of the muscle having a functional level at a predetermined rate.

13. The method according to any of the preceding claims, wherein the three-dimensional water fat separated image is an MRI image.

14. A system (10) configured to determine an effective volume of a muscle of a subject according to the method of any of the claims 1-13, the system comprising
means (12) for acquiring the three-dimensional water and fat separated image, and
means (14) configured to determine the muscle fat fraction level in the muscle, determine the first volume region, and calculate the effective volume.

## Patentansprüche

1. Verfahren (100) zum Quantifizieren eines effektiven Volumens (V_{E}) eines Muskels (20) einer Versuchsperson, wobei das Verfahren folgende Schritte umfasst:
Erfassen (102) eines dreidimensionalen Wasser und Fett trennenden Bildes des Muskels, wobei das Bild Volumenelemente umfasst, die jeweils Wasser- und Fettwerte aufweisen,
Bestimmen (104) eines Muskelfettanteilswertes, MFF1, für jedes Volumenelement in dem erfassten Bild des Muskels,
**dadurch gekennzeichnet, dass** es ferner folgende Schritte umfasst:
Bestimmen (106) einer ersten Volumenregion (30) des Muskels in dem Bild, in welcher der Muskelfettanteilswert, MFF1, unterhalb eines vorbestimmten Schwellenwertes, T1, liegt,
Berechnen (108) einer effektiven Volumendarstellung des Muskels durch Multiplizieren des Volumens der ersten Volumenregion mit WF(MFF1), wobei WF(MFF1) eine Gewichtungsfunktion des Muskelfettanteils, MFF1, in der ersten Volumenregion ist.

2. Verfahren nach Anspruch 1, wobei der vorbestimmte Schwellenwert, T1, des Muskelfettanteils zwischen 30 und 80 %, bevorzugt zwischen 45 und 55 %, weiter bevorzugt bei 50 % liegt.

3. Verfahren nach Anspruch 1 oder 2, wobei die Gewichtungsfunktion WF (MFF1) ferner eine Funktion des vorbestimmten Schwellenwertes, T1, ist.

4. Verfahren nach einem der vorhergehenden Ansprüche, wobei die Gewichtungsfunktion WF (MFF1) gleich 1 ist, wenn MFF1 gleich null ist, und gegen 0 abnimmt, wenn MFF1 gegen T1 zunimmt.

5. Verfahren nach einem der vorhergehenden Ansprüche, wobei die Gewichtungsfunktion WF (MFF1) eine lineare Funktion von MFF1 ist.

6. Verfahren nach Anspruch 4, wobei die Gewichtungsfunktion WF(MFF1) gleich 1-(MFF1/T1) ist.

7. Verfahren nach einem der Ansprüche 1 bis 4, wobei die Gewichtungsfunktion WF(MFF1) eine nicht lineare Funktion von MFF1 ist.

8. Verfahren nach Anspruch 7, wobei die Gewichtungsfunktion WF(MFF1) gleich exp(-k*(MFF1/T1)) ist, wobei k eine positive Ganzzahl ist.

9. Verfahren nach Anspruch 7, wobei die Gewichtungsfunktion WF(MFF1) gleich (1-MFF1/T1)² ist.

10. Verfahren nach einem der vorhergehenden Ansprüche, wobei der Muskelfettanteilswert für jedes Volumenelement basierend auf den Wasser- und Fettwerten für das jeweilige Volumenelement bestimmt wird.

11. Verfahren nach einem der vorhergehenden Ansprüche, wobei die erste Volumenregion (30) durch die Volumenelemente des Muskels (20) in dem Bild bereitgestellt wird, das einen MFF1 unterhalb des vorbestimmten Schwellenwertes, T1, aufweist.

12. Verfahren nach einem der vorhergehenden Ansprüche, wobei das effektive Volumen (30) des Muskels (20) eine Quantifizierung des Teils des Muskels bereitstellt, der einen Funktionswert bei einer vorbestimmten Rate aufweist.

13. Verfahren nach einem der vorhergehenden Ansprüche, wobei das dreidimensionale Wasser und Fett trennende Bild ein MRI-Bild ist.

14. System (10), das dazu konfiguriert ist, ein effektives Volumen eines Muskels einer Versuchsperson gemäß dem Verfahren nach einem der Ansprüche 1 bis 13 zu bestimmen, wobei das System umfasst
Mittel (12) zum Erfassen des dreidimensionalen Wasser und Fett trennenden Bildes, und
Mittel (14), die dazu konfiguriert sind, den Muskelfettanteilswert in dem Muskel zu bestimmen, die erste Volumenregion zu bestimmen und das effektive Volumen zu berechnen.

## Revendications

1. Procédé (100) de quantification d'un volume effectif (V_{E}) d'un muscle (20) d'un sujet, le procédé comprenant les étapes de :
acquérir (102) une image d'eau et de graisse tri-dimensionnelle séparée du muscle, ladite image comprenant des éléments volumiques ayant chacun des valeurs de niveaux d'eau et de graisse,
déterminer (104) un niveau de fraction de graisse musculaire, MFF1, pour chaque élément volumique dans l'image du muscle acquise,
**caractérisé en ce qu'**il comprend en outre les étapes de :
déterminer (106) une première région volumique (30) du muscle dans ladite image dans laquelle le niveau de fraction de graisse musculaire, MFF1, est au-dessous d'un niveau seuil prédéterminé, T1,
calculer (108) une représentation volumique effective du muscle en multipliant le volume de la première région volumique avec W(MFF1), dans lequel W(MFF1) est une fonction de pondération de la fraction de graisse musculaire, MFF1, dans ladite première région volumique.

2. Procédé selon la revendication 1, dans lequel le niveau seuil prédéterminé, T1, de fraction de graisse musculaire est entre 30-80 %, de préférence entre 45-55 %, de manière plus préférée de 50 %.

3. Procédé selon la revendication 1 ou 2, dans lequel la fonction de pondération WF(MFF1) est en outre une fonction du niveau seuil prédéterminé, T1.

4. Procédé selon l'une quelconque des revendications précédentes, dans lequel la fonction de pondération WF(MFF1) est égale à 1 lorsque MFF1 est zéro et décroît vers 0 lorsque MFF1 augmente vers T1.

5. Procédé selon l'une quelconque des revendications précédentes, dans lequel la fonction de pondération WF(MFF1) est une fonction linéaire de MFF1.

6. Procédé selon la revendication 4, dans lequel la fonction de pondération WF(MFF1) est 1-(MFF1/T1).

7. Procédé selon l'une quelconque des revendications 1-4, dans lequel la fonction de pondération WF(MFF1) est une fonction non-linéaire de MFF1.

8. Procédé selon la revendication 7, dans lequel la fonction de pondération WF(MFF1) est exp(-k*(MFF1/T1)), où k est un nombre entier positif.

9. Procédé selon la revendication 7, dans lequel la fonction de pondération WF(MFF1) est (1-(MFF1/T1)².

10. Procédé selon l'une quelconque des revendications précédentes, dans lequel le niveau de fraction de graisse musculaire est déterminé pour chaque élément volumique en se basant sur les valeurs de niveau d'eau et de graisse pour l'élément volumique respectif.

11. Procédé selon l'une quelconque des revendications précédentes, dans lequel la première région volumique (30) est fournie par les éléments volumiques du muscle (20) dans l'image ayant un MFF1 en-dessous du niveau seuil prédéterminé, T1.

12. Procédé selon l'une quelconque des revendications précédentes, dans lequel le volume effectif (30) du muscle (20) fournit une quantification de la partie du muscle ayant un niveau fonctionnel à une vitesse prédéterminée.

13. Procédé selon l'une quelconque des revendications précédentes, dans lequel l'image d'eau et de graisse tri-dimensionnelle séparée est une image d'IRM.

14. Système (10) configuré pour déterminer un volume effectif d'un muscle d'un sujet selon le procédé selon l'une quelconque des revendications 1-13, le système comprenant
un moyen (12) pour acquérir l'image d'eau et de graisse tri-dimensionnelle séparée, et
un moyen (14) configuré pour déterminer le niveau de fraction de graisse musculaire dans le muscle, déterminer la première région volumique et calculer le volume effectif.
